Europäisches Patentamt

European Patent Office

Office européen des brevets

(19)

(11) Publication number: **0 205 720**
**A1**

## EUROPEAN PATENT APPLICATION

(21) Application number: 85850224.8

(22) Date of filing: **28.06.85**

(51) Int. Cl.⁴: **A 61 B 6/03, A 61 N 5/10**

(43) Date of publication of application: **30.12.86**
**Bulletin 86/52**

(84) Designated Contracting States: **AT BE CH DE FR GB IT LI LU NL SE**

(71) Applicant: **INSTRUMENT AB SCANDITRONIX, Husbyborg, S-755 90 Uppsala (SE)**

(72) Inventor: **Brahme, Anders, Stugvägen 40, S-161 46 Bromma (SE)**

(74) Representative: **Svanfeldt, Hans-Ake et al, DR. LUDWIG BRANN PATENTBYRA AB Box 1344 Drottninggatan 7, S-751 43 Uppsala (SE)**

(54) CT scanner for radiation theraphy planning.

(57) A device for making photon CT images with the patient in radiation therapy position. A narrow pencil shaped beam of photons (5) the energy of which is of the order of about 3 up to about 50 MeV is scanned transversally over the patient. During scanning the photons transmitted through the patient's body are measured by the detector array (6). A computer (13) then reconstructs a CT-image of the body from the photon transmission data and the position data of the scanning beam (5). The CT image thus determined is used for planning the patient treatment in a separate dose planning computer (16). The patient is then treated by scanning the beam over the treatment volume and in so doing using the recorded position data to position the beam and the absorbtion data to vary the scan pattern of the beam such that the desired dose distribution in the tumor volume is obtained.

A CT SCANNER FOR RADIATION TERAPHY

The present invention relates to a method and device of making photon CT images with the patient in the radiation teraphy position by subjecting the patient to the peraphy radiation beam. The photon radiation used for taking the CT images is also used for the teraphy radiation. At first the CT images are taken and are computer processed and then, some minutes later while the patient still is resting on the patient couch, the radiation teraphy is performed. This method is generally known from Med.Phys.9(4), July/Aug 1982, Am. Asoc. Phys. Med. wherein R.G. Siompson et al. in a technical report entitled "A 4-MV CT scanner for radiation teraphy: The prototype system." is describing the novel concept referred to above.

In the known system a flat fan like photon beam is directed towards the partient and a photon detector array, placed under the patient's couch, is providing electrical signals representing the photon transmission along a sectional view of the patient's body. The gantry, which provides the fan like photon beam, is rotated in small steps around the patient's body and for each gantry setting the electrical signals are stored in a computer which out of the date so collected forms a CT image of the sectional view of the patient. The main advantage with this known concept is that no separate diagnostic-energy CT scanner is required. Morover, the contrast to the images obtained with the known 4-MV CT scanner is improved, particularly the contrast to body tissue, Also the dose planning is more accurate. Previously the attenuation coefficients of the tissue, taken with diagnostic radiation energy of typically 70 keV has to be extrapolated to those expected to be present at the tereaphy radiation energies, which typically may be in the order of abut 5 MeV. An estrapolation over such a wide range is uncertain.

Before the Simpson consept was desired conventinally the teraphy treatment was preceded by an X-ray screening - performed by a conventional CT-scanner - in order to localize the tumor volume. Thereafter the patient was moved to the couch of a teraphy apparatus the radiation field of which was set (by a collimator) on basis of the CT-image. Apparently the coordinate system of the teraphy apparatus is different from what of the CT-scanner making it difficult to exactly position the teraphy apparatus on the tumor volume. With the Simpson concept such positioning arrows of the tumor volume are obviated.

The Simpson CT-scanner referred to above have some inherent drawbacks among which the following are apparent:

- The flat, broad fan like beam radiates a "slice" of the patient's body simultaneously implying that a detector included in the detector ray under the patent's couch further to electrones which pass the volyme immediately above the detector and secondary electrones generated by said volume also will detect secondary electrones generated in the volume surrounding said volume immedately above the said detector. This degrades the resolution of the final CT-image.

- Dynamic radiation treatment of the tumor volume to the desired dose rate is difficult to perform. While rotating the gantry of the treatment apparatus the field collimator must be continuously readjusted to adapt its field to the shape of the tumor volume as seen from the gantry.

- The radiation is of a specific energy (4 MeV). Should a different energy be used then a different beam flattening filter must be used. Such beam flattening filters further to being large and heavy unevitable absorbs the high energy electrones included in the primary beam from the accelerator.

The present invention is directed to a method and apparatus for reducing the deficiences inherent in the known Simpson CT-scanner, particularly:

- The resolution of the CT-image should be improved, - dynamic radiation treatment should be possible to perform in an easy way, - a spectrum of different radiation energies should be available thereby making it possible to take CT-images at a desired beam energy without using beam flattening filters which inevitable absorbs the high energy electrones, that is exactly those electrones which in accordance with the present invention should be used to make the CT images.

The above objects of the invention are achieved with a method and device as defined in the accompanying patent claims.

A preferred embodiment of the invention should be described in conjunction with the accompanying drawings wherein:

Fig. 1 is a diagram showing the absorbtion cross section for diferent tissues versus the energy of the photon beam,

Fig. 2 is an end view of the apparatus in accordance with the present invention,

Fig. 3 schematically shows the electrical signal obtained at the photon detector array for the specific position (Fig. 3a) of the scanned beam in accordance with the present invention, and for the next following position (Fig. 3b) of the scanning beam,

Fig. 4 is a corresponding electrical signal obtained at the photon detector of the known Simpson device and this figure has been incorporated in order to illustrate the differences between the known device and that of the present invention,

Fig. 5 illustrates the format in which position and photon transmission data are stored in the memory of a micro computer, and

Fig. 6 illustrates a block diagram of the apparatus in accordance with the present invention.

4

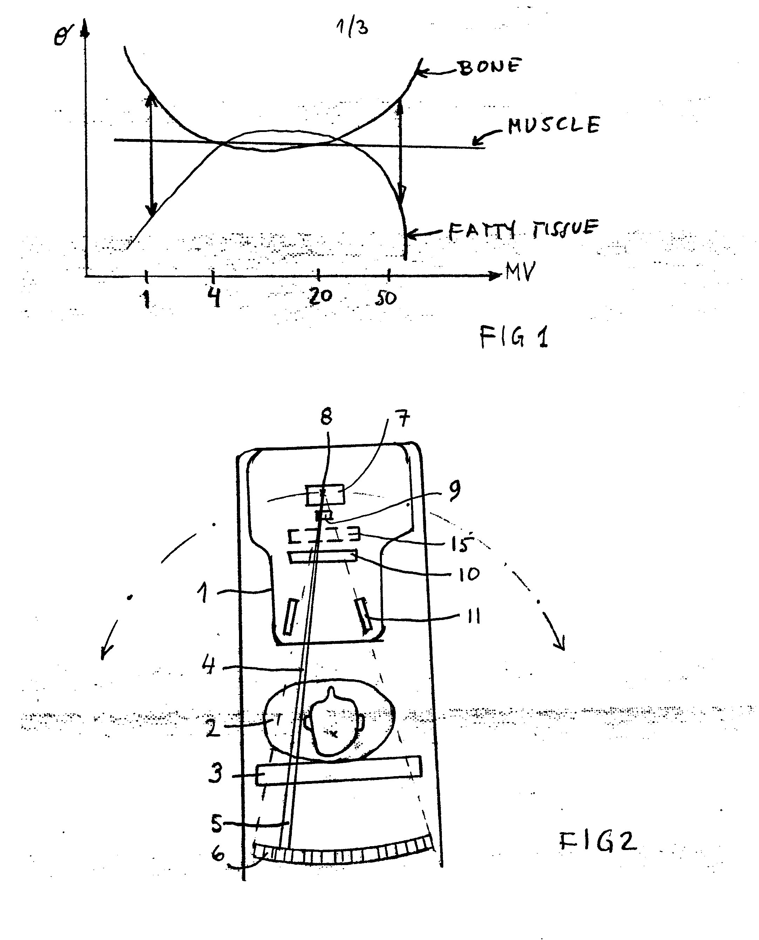

In Fig. 1 the absorption curves for bone, muscles and fatty tissues respectively are shown for varying photon energies. When the difference between the attanuation of the radiation in e.g. bone and fatty tissues is large, indicated with vertical double edged arrows, then the contrast of the CT image taken at the indicated radiation energi, is large. The larger the contrast the better the image quality. From Fig. 1 and the left vertical arrow it is apparent why conventional CT scanners are operating with an acceleration voltage of less than about 1 MV. From the right vertical arrow in Fig. 1 it is also apparent that for radiation energies over about 50 MV the contrast is high. The Simpson concept referred to above did take advantage of this fact. Moreover, the dose planning, as suggested by Simpson, was more accurate since the CT image taken with this high energy radiation showed exactly how the organs within the body absorbed the radiation and from this CT image it was possible to compute the real attenuation coefficients of the organs without relying on extrapolation from 70 kV CT images to teraphy radiation energies in the order of 4 MV and above. Such extrapolation is commonly done with the aid of curves similar to that shown in Fig. 1.

In Fig. 2 there is shown a principal set up of the apparatus in accordance with the present invention. From a gantry 1 a patient 2 resting on a patient's couch 3 is radiated with a pencil like, well united, beam 4 of photons, some of which 5, penetrates the body and are detected by a detector array 6.

A proton beam generated by an accelerator not shown, e.g. a race track microtron, enters the gantry 1 which comprises a pair of scanning electromagnets 7 which are energized by a varuing dc-current in order to scan the proton beam in the plane of the drawing between the end of positions shown in broken lines. The scanning system is preferably of the kind described in our U.S. Patent Serial No. 4 442 352. Generally at the scanning centre 8 of the proton beam there is a target 9 of tantal or other metarial known per se which produces photons of generally the same energy as that of the proton

5

beam. Further down in the gantry there is a transmission ion chamber 10 measuring the beam current and a pair of collimator blocks 11 for beam collimation.

The beam is pulsed which means it comprises pulses of baout 1-5 u duration with a repitition frequency of 50-250 MHz. Due to the pulse nature of the beam the detector array is of integrating type, that is rather than counting each photon requiring a very fast responding detector it integrates the number of photons which are detected over a time period. The detector array comprises a linear array of a number of Bitmuthgermante crystals arranged in a side by side relationship and each optically coupled to a photodiod which delivers an electrical signal proportional to the said intergral, which in turn therefor is proportional to the transmission of the radiated body tissue.

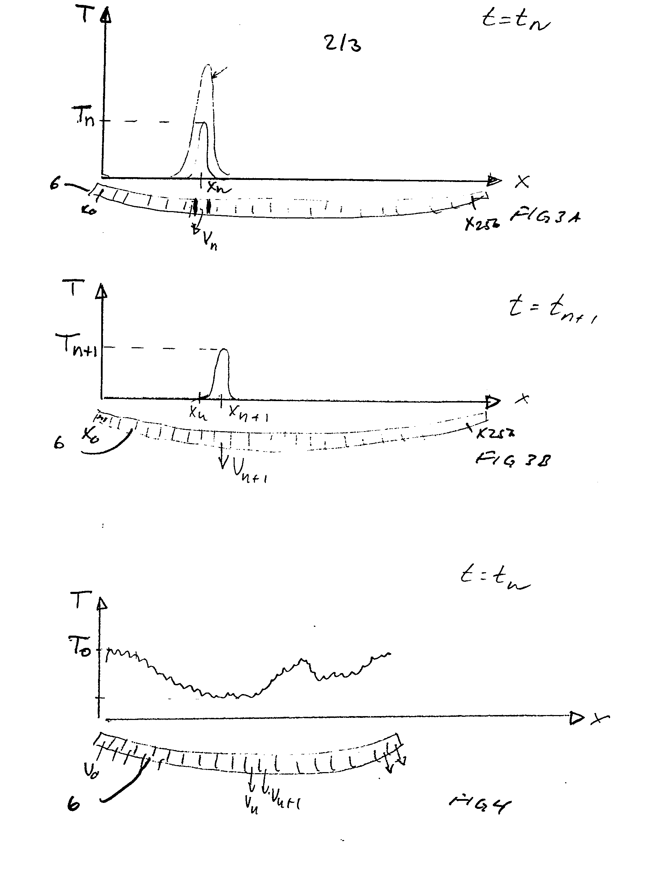

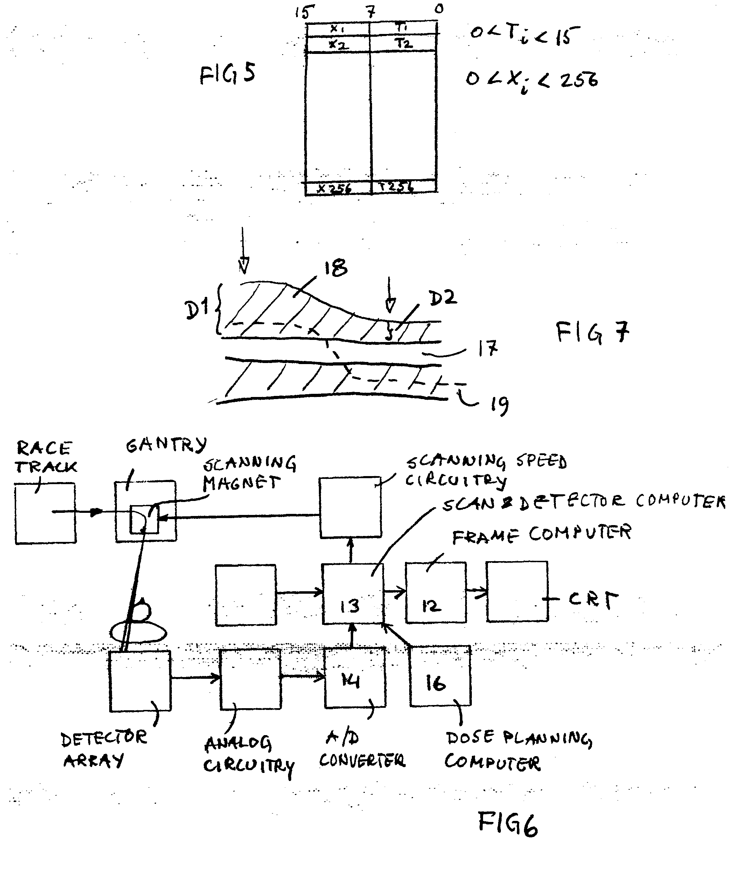

In Figure 3A there is shown the detector array 6 and its separate detectors $x_i$. In the shown embodiment the physical length of the detector is about 60 cm comprising 128 detectors each made of a crystal Bismuthgermanete allowing for a picture resolution about 5 mm which a beam which is about 2 cm in square. When the beam is at crystal $x_n$ at time $t_n$ the photon transmission has a value of $T_n$. In Figure 3B the beam has moved to $x_{n+1}$ at time $t_{n+1}$ and the transmission is $T_{n+1}$. The transmission values are arranged within a grey scale comprise 16 grey levels 0 corresponding to white and 16 to black. From the above it is clear that the linear array 6 is used as a coordinate system for the beam. For each position $x_i$ there is a transmission value $T_i$ which is allocated $x_i$. The position and transmission data for each scan of the beam over the array is recorded in a micro computer which preferably is of the 16 bit type; allowing convenient format for storing said data. This format is shown in Figure 5 from which it appears that high oder byte stores the position of the beam while the low order byte stores the corresponding transmission values. After each scan of the beam over the array the gantry is rotated together with the array thrugh a predetermined angel around the patient's couch which is at rest and a new scan is made and new position and transmission data values are recorded. This is repeated for each angular setting of the gantry until finally a completed 360° rotation of the gantry has been made. The data so collected and recorded is processed in a manner known per se in a frame computer shown at 12 in Figure 6 in order to reconstruct a CT image of the "slice" of the patient's body just irradiated. Thereafter either the patient's couch or the beam or both are moved a predetermied distance in the longitudinal direction of the couch and a new CT image of the body is taken while rotating the gantry around the patient's body.

Instead of assigning a transmission value $T_i$ to each of the positions $x_i$ by taking a separate reading at each detector which for example is done by using a multiplexer the output of which is connected to a scan and detector comupter 13 shown in Figure 6 connected to a common output conductor via an A/D converter 14, the outputs of each of the detectors in the array one connected to a common conductor connected to said scan and detector computer 13; said common conductor in this case carrying a composite signal of thekind indicated in Figure 4.

For reference and for monitoring the incident photon beam a second photon detector 15 is inserted between the target 9 and the transmission ion chamber 10. Should the photon fluence vary during the scanning, the transmission values are correspondingly modified.

In accordance with the present invention the energy of the photons should be in the order of 50 Mev in order to produce pair production which gives a significant improvement in the contrast of the CT image.

From the quotient of the incident photon fluence to the recorded transmission value the attenuation cofficient is determined for each position $x_i$ of the beam. This is made in a

0205720

7

separate dose planning computer 16. The tumor is identified either by inspection of the CT-images taken by the apparatus in accordance with the present invention or by inspection of CT-images taken by a conventioneal (70-140 kV) CT-scanner. In both cases the dose is then planned with the aid of the dose planning computer 16.

If the dose is planned with the aid of a conventional CT-scanner image then the latter image is copied within the frame computer on the photon CT-image taken with the apparatus in accordance with the present invention. The resultant image will thus contain - for each pixel - (picture element of the image)

1) The position of each pixel (and therefore the position of the corresponding part of the patient's organ/tissue) expressed in the coordinate system of the CT scanner in accordance with the present invention and

2) the absorbtion coefficient of the corresponding part of the patient's organ(tissue, that is how much of the irradiation beam that is absorbed in different parts of the patent's body.

This will improve the dose planning since no extrapolation of the kind referred to above is needed. Moreover, no positioning errors will encounter. The dose planning is a matter of minutes and during this time the patient remains resting at the couch.

The radiation may also very easily perform dynamic radiation treatment of the target volume. In Figure 7 the target volume, for example a part of the throat of a patient, is shown at 17. The target volume should be treated with a constant dose rate. The overlying tissue, shown at 18, has varing thickness (shown at D1 och D2 respectively).

The incident radiation, shown at the vertical arrows, will therefore be absorbed in different rates in the tissue 13 leading to an uneven dose rate in the target volume 17. The broken line 19 indicates the position of the dose maximum should the target 17 be radiated with a constant dose. As appears the maximum dose is situated beneath the target volume 17 when the position D2 is radiated from above. This is unwanted, since you want the dose maximum to lye at the same lavel as in portion D1. To compensate for this a smaller dose should be given in the thinner dissue D2 than in the thicker D1. By varying the speed of the scanning beam so that it is residing a longer time in the thicker tissue the dose rate in the target volume 17 is made constant over the target volume. For how long the beam should reside for example in the tissue is determined from the compuded absorbation coefficient for the coresponding portion of the thicker tissue and is made under program control from the dose planning computer.

CLAIMS

1. A method of making photon CT images with the patient in radiation teraphy position by subjecting the patient to the teraphy position by subjecting the patient to the teraphy radiation beam, comprising the steps of:

- scanning a narrow pencil shaped beam of photons or neutrons the energy of which is of the order of about 3 up to about 50 MeV transversly over the patient,

- during said scanning measuring and recording - with a photon detector array located under the patient - the photons transmitted through the patient's body,

- allocating the current position of the scanning beam to the photon transmission - as measured - at said position,

- processing the date so recorded in order to form a photon CT image comprising the photon absorbtion properties of the patient's body at the photon energy used,

- using the photon absobtion data CT image thus determined for planning the patient treatment,

- irraditating the patient by scanning the beam over the treatment volume and in so doing using the recorded position data to position the beam and said absorbtion data to vary the scan pattern of the beam such that the desired dose distribution in the tumor volume is obtained.

2. A method in accordance with claim 1, characterized by copying the absorbtion data CT image on an image formed by conventional CT apparatus and during said copy using the coordiate system of the recorded position data of the absorbtion data CT image to obtain occurate absorbtion data of each point of the tumor to be irradiated, and using the information so obtained during the scanning of the treatment beam to allocate each point of the tumor volume a desired irradiation dose.

3. A method in accordance with claim 2, characterized in that said scan pattern is varied by varying the speed of which the beam is scanned over the tumor volume during the radiation teraphy.

4.  A device for making photon CT image with the patient in the radiation theraphy position by subjecting the patient to the teraphy radiation, comprising:
- an accelerator producing a beam of high energy electrons,
- an electromagnetically operated beam scanning system for scanning the beam at least in a teransverse direction over the patient's body,
- a target for the electron beam, said target being arranged generally at the scanning centre of the scanning system so as to produce a narrow, pencil formed beam of photons,
- a photon detector array positioned under the patient's body,
- first signal processor means connected to the photon detector array for producing first electrical signals representing the photons transmitted through the target volume at each current position of the scanned photon beam,
- second signal processor means for producing second electrical signals representing the current position of the scanning beam,
- computer means to store data relating to the photon attenuation of the target volume and obtained from said first electrical signals, to store data realting to the position said beam had when each first signal was generated,
- and frame computer means for planning the dose at each position of the target volume using said attenuation and position data and for controlling the movement and the speed of the scanned beam during teraphy radiation by using said dose data and said previously stored position data.

5.  A device in accordance with claim 4, characterized in that the energy of the photons is in the order of about 3 MeV up to about 50 MeV.

6.  A device in accordance with claim 4, characterized in that the photon detector array comprises bitmuthgermanate crystals arranged in a side by side relationship along a curved surface the centre of curvature of which coincides with

11

the scanning center of the scanning system.

7. A device in accordance with claim 6, characterized by means for moving the detector array in the longitudial direction of the patient.

8. A device in accordance with claim 4, characterized by the fact that the second signal processor means comprise current sensors connected to an ion transmission chamber inserted between the target and a collimator included in the gantry which also houses the scanning magnets of the scanning system for the beam.

9. A device in accordance with claim 4, characterized in that the energy of the photons is high enough to produce pair generation effect in the tissue of the tumor volume.

FIG 1

FIG2

0205720

2/3

$t = t_n$

$T_n$

$X_n$

$V_n$

$X_0$

$X_{256}$

FIG 3A

$t = t_{n+1}$

$T_{n+1}$

$X_n$   $X_{n+1}$

$X_0$

$X_{256}$

$V_{n+1}$

FIG 3B

$t = t_n$

$T_0$

$V_0$

$V_n$  $V_{n+1}$

FIG 4

0205720

3/3

FIG 5

$0 < T_i < 15$

$0 < X_i < 256$

FIG 7

FIG 6

## PARTIAL EUROPEAN SEARCH REPORT

European Patent Office

which under Rule 45 of the European Patent Convention shall be considered, for the purposes of subsequent proceedings, as the European search report

Application number

EP 85 85 0224

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| D,Y | MEDICAL PHYSICS, vol.9, no.4, July/August 1982, pages 574-579; New York, US R.G. SIMPSON et al.: "A 4-MV CT scanner for radiation therapy: The prototype system." | | A 61 B 6/03 A 61 N 5/10 |
| | * Whole article * | 1,4 | |
| A | | 3 | |
| | --- | | |
| Y | BROWN BOVERI REVIEW, vol.71, no.5, May 1984, pages 245-250; Baden, CH H. VOGT: "Linear accelerator DYNARAY-CH20." | | |
| | * Whole article * | 1,4 | |
| A | | 5,8 | |
| | --- | | |
| A | US-A- 4 118 631 (FROGGATT) | | |
| | * Figures 1-4; column 2, line 8 - column 4, line 55 * | 1,2 | |

TECHNICAL FIELDS SEARCHED (Int. Cl.4)

A 61 B
A 61 N

## INCOMPLETE SEARCH        ---        ./.

The Search Division considers that the present European patent application does not comply with the provisions of the European Patent Convention to such an extent that it is not possible to carry out a meaningful search into the state of the art on the basis of some of the claims.

Claims searched completely:   4 – 9

Claims searched incompletely:  1 – 3

Claims not searched:

Reason for the limitation of the search:

Method for treatment of the human or animal body by surgery or therapy (see art. 52(4) of the European Patent Convention).

| Place of search | Date of completion of the search | Examiner |
|---|---|---|
| The Hague | 28-02-1986 | CHEN |

EPO Form 1505.1 03.82

| | DOCUMENTS CONSIDERED TO BE RELEVANT | | CLASSIFICATION OF THE APPLICATION (Int. Cl.4) |
|---|---|---|---|
| Category | Citation of document with indication, where appropriate, of relevant passages | Relevant to claim | |
| A | MEDICAL PHYSICS, vol.10, no.3, May/ June 1983, pages 347-351; New York, US<br>W. SWINDELL: "A 4-MV CT scanner for radiation therapy; spectral properties of the therapy beam."<br><br>* Abstract; page 348, figure 1 *<br>--- | 4,6 | |
| A | US-A- 4 045 672 (WATANABE)<br><br>* Figures 1,2,5; column 1, line 65 - column 4, line 7 *<br>------- | 4,7 | TECHNICAL FIELDS SEARCHED (Int. Cl.4) |